# EUROPÄISCHE PATENTSCHRIFT

(11) **EP 1 788 076 B1**
(45) Veröffentlichungstag und Bekanntmachung des Hinweises auf die Patenterteilung: **21.01.2009**
(21) Anmeldenummer: 06023193.3
(22) Anmeldetag: 08.11.2006
(51) Int. Cl.: C12N 5/00

(54) **Verwendung von Estern ungesättigter, physiologisch aktiver Fettsäuren als Nährmedien für Zellkulturen**
Use of esters of unsaturated physiologically active fatty acids as nutrient media for cell cultures
Utilisation des esters des acides gras insaturés physiologiquement actifs en tant que médias nutritifs pour des cultures de cellules

(30) Priorität: 16.11.2005 DE 102005054577
(43) Veröffentlichungstag der Anmeldung: 23.05.2007
(73) Patentinhaber: Cognis IP Management GmbH, 40589 Düsseldorf (DE)
(72) Erfinder: Strube, Albert, 41470 Neuss (DE); Scholz, Claudia, 14167 Berlin (DE); Seifert, Jochen, 64367 Mühltal (DE); Carité, Christophe, 87570 Rilhac-Rancon (FR); Sicre, Christophe, 31800 Landorthe (FR); Eychenne, Valerie, 31400 Toulouse (FR)

(56) Entgegenhaltungen:
- EP-A2- 0 060 565
- WO-A-00/27996
- US-A- 4 786 599
- US-A- 5 266 479

## Beschreibung

### Gebiet der Erfindung

Die Erfindung befindet sich auf dem Gebiet der Zellkultivierung und betrifft die Verwendung von speziellen Fettsäureniedrigalkylestern als Nährmedien für Zellkulturen und insbesondere als Ersatz für fötales Tierserum.

### Stand der Technik

Fötales Tierserum (Foetal Bovine Serum = FBS) ist wegen seiner großen Anwendungsbreite das verbreitetste Serum zur *in-vitro* Kultivierung von Zellen, Geweben und Organen, das sowohl in der Industrie als auch in Medizin und Wissenschaft Verwendung findet. Weltweit liegt der Bedarf - der fast ausschließlich durch fötales Kalbsserum gedeckt wird - bei etwa 500.000 Litern pro Jahr.

Das Blut von den Tierföten, die für FBS Produktion benutzt werden, wird normalerweise durch Durchbohren der Nabelschnur oder der Jugularader gewonnen, vorzugsweise wird aber aus septischen Gründen das Herz punktiert. Obschon in der Fachwelt die Auffassung weit verbreitet ist, dass der Fötus ungefähr zur gleichen Zeit wie das Muttertier verstirbt, ist nicht auszuschließen, dass auch nach dem Zeitpunkt, an dem das Herz des Muttertieres zu schlagen aufgehört hat, der Fötus noch eine ganze Weile weiter lebt, da wissenschaftliche Untersuchungen gezeigt haben, dass Föten und Neugeborene Sauerstoffmangel vergleichsweise gut verkraften können. Ebenfalls nicht auszuschließen ist, dass der Fötus bereits über ein Schmerzempfinden verfügt, was den Vorgang der Gewinnung von FBV zu einer aus ethischen Überlegungen zweifelhaften Praxis macht.

Die Aufgabe der vorliegenden Erfindung hat somit darin bestanden, im Sinne von Nachhaltigkeit und Tierschutz ein Nährmedium für eine große Zahl sehr unterschiedlicher Zelllinien mit hinreichender Löslichkeit in den Kulturen zu entwickeln, dass in seinen Eigenschaften Serum tierischer Herkunft vergleichbar ist, jedoch auf synthetischem Wege, vorzugsweise auf Basis pflanzlicher oder mariner Ausgangsstoffe gewonnen wird und damit die Gewinnung FBS mittelfristig überflüssig macht.

US-A 4,762,792 offenbart ein wässriges Lipoproteinkonzentrat auf tierischer Basis.

### Beschreibung der Erfindung

Gegenstand der Erfindung ist die Verwendung von Estern ungesättigter, physiologisch aktiver Fettsäuren als Nährmedien für die Zellkultivierung, speziell als Ersatz für Fötales Tierserum.

Überraschenderweise wurde gefunden, dass die erfindungsgemäß zu verwendenden Ester ausgezeichnete Nähreigenschaften aufweisen, wie z.B. in Tests an Zelllinien des Typs "BHK" (Fibroblasten von Hamsternieren), "Vero" (Fibroblasten von Grünaffennieren) oder "MRC5" (Fibroblasten aus menschlichem Lungengewebe) gezeigt werden konnte und sich dabei FBS als gleichwertig oder sogar überlegen erweisen.

### Ungesättigte,physiologisch aktive Fettsäureester

Ein gemeinsames Kriterium der im Sinne der erfindungsgemäß einzusetzenden physiologisch aktiven Fettsäureester besteht darin, dass sie über einen hinreichend langen Lipidrest und eine ausreichende Zahl von Doppelbindungen verfügen. Für diesen Zweck eignen sich daher insbesondere Ester solcher Fettsäuren, die 16 bis 26 Kohlenstoffatome und 2 bis 6 Doppelbindungen aufweisen.

In einer ersten Ausführungsform werden für diesen Zweck Ester der konjugierten Linolsäure (CLA), speziell solche mit niederen aliphatischen Alkoholen mit 1 bis 4 Kohlenstoffatomen und besonders bevorzugt deren Ethylester eingesetzt. Dabei handelt es sich um bekannte Stoffe, die beispielsweise durch basenkatalysierte Isomerisierung von Distelöl oder entsprechenden Alkylestern, nachfolgende enzymatische Hydrolyse sowie Veresterung hergestellt werden können. Es hat sich dabei als vorteilhaft erwiesen, wenn die CLA-Komponente eine bestimmte Spezifikation erfüllt, gemäß der der Acylrest wenigstens 30 Gew.-% t10, c12-Isomere, wenigstens 30 Gew.-% c9,t11-Isomere und in Summe weniger als 1 Gew.-% 8,10-, 11,13- und t,t-Isomere aufweist. Entsprechende Ausgangssäuren sind beispielsweise unter der Bezeichnung Tonalin® CLA-80 (Cognis) im Handel.

In einer zweiten alternativen Ausführungsform kommen auch Ester so genannter omega-Fettsäuren, und speziell omega-3-Fettsäuren in Frage, die typisch 18 bis 26 und insbesondere 20 bis 22 Kohlenstoffatome enthalten und dabei wenigstens 4, bis hin zu 6 Doppelbindungen aufweisen. Auch solche Stoffe sind nach üblichen Methoden der organischen Chemie erhältlich, beispielsweise durch Umesterung von Fischöl, Harnstofffällung der erhaltenen C₁-C₄-Alkylester und nachfolgende Extraktion mit unpolaren Lösemitteln, wie beschrieben in der deutschen Patentschrift DE 3926658 C2 (Norsk Hydro). Auf diese Weise werden Gemische von Fettsäureestern erhalten, die reich an omega-3 (all-Z)-5,8,11,14,17-eicosapentansäure (EPA) C20:5 und (all-Z)-4,7,10,13,16,19-Docosahexansäure (DHA) C22:6. sind. Solche Ausgangsfettsäuren sind beispielsweise unter der Bezeichnung Omacor® (Pronova) im Handel. Alternativ können Öle mit einem vergleichbar hohen Gehalt an EPA und DHA auch auf mikrobiellem Wege hergestellt werden, nämlich durch Fermentation bestimmter Pilze oder Mikroalgen, wie z.B. beschrieben in EP 0515460 B1 (Martek) oder WO 98/003671 A1 (Santory).

Die physiologisch aktiven Komponenten können jedoch nicht nur in Form ihrer Niedrigalkylester, sondern auch ihrer Ester mit Sterolen eingesetzt werden. Die Sterolester haben den Vorteil, dass sie leicht resorbiert und unter physiologischen Bedingungen ebenfalls leicht gespalten werden. Zudem wird mit dem Sterol ein weiterer physiologisch aktiver Wirkstoff freigesetzt. Die Begriffe "Sterol" "Stanol" und "Sterin" sind dabei synonym zu verwenden und bezeichnen Steroide, die nur am C-3 eine Hydroxylgruppe und ansonsten keine Funktionen aufweisen. Zusätzlich können die 27 bis 30 Kohlenstoffatome umfassenden Sterole eine Doppelbindung, vorzugsweise in 5/6-Stellung enthalten; typische Beispiele sind die Ester mit β-Sitosterol, Campesterol, Brassicasterol, Avenasterol und Stigmasterol. Die im Sinne der Erfindung bevorzugten Verbindungen sind die Ester der CLA oder der omega-Fettsäuren mit β-Sitosterol oder dessen Hydrierungsprodukt, dem β-Sitostanol.

In einer bevorzugten Ausführungsform werden als physiologisch aktiven Komponenten Umesterungsprodukte aus einem natürlichen oder synthetischen Öl oder einer Ölmischung mit einem hohen Gehalt, vorzugsweise von mehr als 50 und insbesondere mehr al 75 Mol-% an ungesättigten, physiologisch aktiven Fettsäuren - bezogen auf die Acylreste - und einem niederen C₁-C₄-Alkohol, vorzugsweise Ethanol, oder einem Sterol eingesetzt. Die Ausgangsöle oder Ölgemische sind dabei vorzugsweise pflanzlicher, mariner oder mikrobieller Natur und können auch Mischungen dieser Stoffe darstellen. Typische Beispiele hierfür sind Diestelöl, Sonnenblumenöl, Rapsöl, Olivenöl, Leinöl, Baumwollsaatöl, Reisöl, Makrelenöl, Sardinenöl, Heringsöl sowie die schon eingangs genannten mikrobiellen Öle aus der Fermentation von marinen Mikroorganismen. Die Umesterung dieser Öle kann in an sich bekannter Weise durchgeführt werden, d.h. gegebenenfalls in Gegenwart eines alkalischen Katalysators, ohne dass hierzu der Fachmann in erfinderischer Weise tätig werden muss.

In einer weiteren bevorzugten Ausführungsform können die Ester der ungesättigten, physiologisch aktiven Fettsäuren mit Sterolen abgemischt werden. Auch hier sind als typische Beispiele für geeignete Sterole Campesterol, Brassicasterol, Avenasterol, Stigmasterol und insbesondere β-Sitosterol zu nennen. Das Mischungsverhältnis kann dabei 99 : 1 bis 50 : 50, vorzugsweise 98 : 2 bis 75 : 25 und insbesondere 95 : 5 bis 90 : 10 Gewichtsteile betragen. Besonders bevorzugt sind Mischungen von CLA-Ethylester und β-Sitosterol, insbesondere im Gewichtsverhältnis 99 : 1 bis 96 : 4. Der Vorteil bei der Verwendung dieser Mischungen liegt in besseren Kultivierungsergebnissen bei speziellen Zelllinien, z.B. bei Epithelzellen von Hamsterovarien (CHO).

Zur Verbesserung der Löslichkeit der erfindungsgemäß zu verwendenden Fettsäureester hat es sich als vorteilhaft erwiesen, Phospholipide einzusetzen. Hierzu können die Fettsäureester entweder mit den Phospholipiden, welche vorzugsweise die gleichen Acylreste aufweisen, im Gewichtsverhältnis insbesondere von 20 : 80 bis 80 : 20 und vorzugsweise 40 : 60 bis 60 : 40 abgemischt oder aber in an sich bekannter Weise liposomal verkapselt werden. Werden Liposomen eingesetzt, können diese in der Membran zusätzlich pflanzliche Proteine eingelagert enthalten, welche sich beispielsweise von Soja oder Weizen ableiten.

### Beispiele

### Belspiel 1

### Herstellung eines ungesättigten Fettsäureethylesters

Eine Mischung bestehend aus 20 Gew.-% Palmöl, 5 Gew.-% Rapsöl (alte Züchtung), 50 Gew.-% Rapsöl mit hohem Erucasäuregehalt, 20 Gew.-% Sonnenblumenöl und 5 Gew.-% Leinöl wurde in an sich bekannter Weise einer Umesterung mit Ethanol unterworfen. Das resultierende Ethylestergemisch zeigte die folgende Fettsäureverteilung:

| | |
|---|---|
| C12: | 0,07 Gew.-% |
| C14: | 0,31 Gew.-% |
| C16:0: | 14,64 Gew.-% |
| C16:1 omega-7: | 0,15 Gew.-% |
| C18:0: | 2,50 Gew.-% |
| C18:1 omega-7: | 21,48 Gew.-% |
| C18:2 omega-6: | 23,26 Gew.-% |
| C18:3 omega-3: | 7,38 Gew.-% |
| C20:0: | 0,52 Gew.-% |
| C20:1: | 3,38 Gew.-% |
| C22:1: | 21,71 Gew.-% |

## Patentansprüche

1. Verwendung von Estern von Fettsäuren mit 16 bis 24 Kohlenstoffatomen und 2 bis 5 Doppelbindungen als Nährmedien für die Zellkultivierung, **dadurch gekennzeichnet, dass** man die Fettsäuren in Form ihrer Ester mit Sterolen einsetzt.

## Claims

1. Use of esters of fatty acids containing 16 to 24 carbon atoms and 2 to 5 double bonds as nutrient media for cell cultivation, **characterized in that** the fatty acids are used in the form of their esters with sterols.

## Revendications

1. Utilisation d'esters d'acides gras renfermant de 16 à 24 atomes de carbone et de 2 à 5 doubles liaisons en tant que milieux nutritifs pour la culture de cellules, **caractérisée en ce que** les acides gras sont utilisés sous la forme de leurs esters avec des stérols.
